# EUROPÄISCHE PATENTSCHRIFT

(11) **EP 0 292 695 B1**
(45) Veröffentlichungstag und Bekanntmachung des Hinweises auf die Patenterteilung: **17.02.1993**
(21) Anmeldenummer: 88106291.3
(22) Anmeldetag: 20.04.1988
(51) Int. Cl.: A61N 5/06, G02B 6/24

(54) **Einrichtung zur zirkumferenziellen Bestrahlung von Objekten**
Device for circumferential irradiation of objects
Dispositif d'irradiation circonférentielle d'objets

(30) Priorität: 25.05.1987 DE 3717525
(43) Veröffentlichungstag der Anmeldung: 30.11.1988
(73) Patentinhaber: Deutsche Aerospace AG, 81663 München (DE)
(72) Erfinder: Hessel, Stefan, Dr., D-8000 München 81 (DE); Ischinger, Thomas, Dr., D-8000 München 81 (DE)

(56) Entgegenhaltungen:
- EP-A- 0 178 464
- EP-A- 0 236 867
- US-A- 4 336 809
- PATENT ABSTRACTS OF JAPAN, Band 4, Nr. 115 (P-23)[597], 16. August 1980; & JP-A-55 69 115 (MITSUBISHI DENKI K.K.) 24-05-1980
- PHYSICS IN MEDICINE & BIOLOGY, Band 29, Nr. 1, Januar 1984, Seiten 53-56, The Institute of Physics, Bristol, GB; A.L. McKENZIE et al.: "How to control beam profile during laser photoradiation therapy"
- "Handbook of Optics" Eds W E Driscoll and W Vaughan, s.13-7 bis 13-8, McGraw-Hill, New York (1978)

## Beschreibung

Die Erfindung betrifft eine Einrichtung zur zirkumferenziellen medizinischen Bestrahlung von Objekten, insbesondere von Gefäßen, Hohlorganen und soliden Geweben mittels optischer Strahlung hoher Intensität, welche über eine flexible Lichtleitfaser geführt wird, in die die optische Strahlung unter einem vorgegebenen Winkel zur Flächennormalen der Stirnfläche der Lichtleitfaser eingekoppelt wird derart, daß sie am distalen Ende der Lichtleitfaser in Form eines Kegelmantels gerichtet austritt.

Aus "Physics in Medicine and Biology", Vol. 29 (1984) S. 53-56 ist eine Einrichtung zur therapeutischen Behandlung von Tumoren mittels Laserbestrahlung bekannt, bei der zur Vergrößerung des aus dem Lichtleiter austretenden Strahlenbündels der Laserstrahl parallel, aber nicht koaxial zur optischen Achse des Lichtleiters verläuft und quasi schräg in diesen eingekoppelt wird. Der aus dem Lichtleiter austretende Strahl weist dann nicht mehr das Gaußsche Strahlprofil des Lasers, sondern ein verbreitertes, nahezu rechteckiges Profil auf. Damit soll eine flächenhafte Bestrahlung des gesamten Tumors ermöglicht werden.

Aus US-A-4 336 809 ist bekannt, daß der aus einem Lichtleiter austretende Strahlungskegel aufgeweitet werden kann, indem das Verhältnis der Brechungsindizes von Lichtleitfaser und Ummantelung so gewählt wird, daß die Innenreflexion am Ende des Faserbündels erheblich geringer als bei Totalreflexion ist.

In "Laser in Surgery and Medicine" 6: 150 bis 154 (1986) sowie in der EP 01 52 766 sind ähnliche Bestrahlungsinstrumente beschrieben ,wobei von hoher Intensität der optischen Strahlung dann gesprochen wird, wenn diese nicht zu Beleuchtungszwecken, sondern zur Koagulation oder Abtragung von Geweben dient. In der erstgenannten Schrift wird eine allseitige Abstrahlung von Laserlicht dadurch bewirkt, daß über das distale Ende einer Lichtleitfaser eine zylindrische, die Faser verlängernde Glaskappe gestülpt wird, deren Innenwand mit einem weißen Kunststoff beschichtet und deren Außenwand durch Sandstrahlen aufgeraut wird. Durch diese Maßnahmen wird eine möglichst hohe Lichtstreuung des aus dem Faserende im wesentlichen axial austretenden Laserlichtes bewirkt, so daß eine diffuse radiale Abstrahlung im Bereich der Kappe erzielt wird. Die in der o.g. EP 01 52 766 beschriebene Einrichtung unterscheidet sich demgegenüber darin, daß auf die Verwendung einer Kappe verzichtet wird und stattdessen das distale Ende der Lichtleitfaser von der Ummantelung befreit wird. Auch damit soll eine im wesentlichen diffuse radiale Abstrahlung des Laserlichts erzielt werden.

Nachteilig bei den o.g. Einrichtungen ist, daß durch die diffuse Abstrahlung die Intensität erheblich reduziert wird, so daß die eingekoppelte Strahlungsleistung zu Koagulations- oder Abtragungszwecken von Geweben, Stenosen und dergleichen entsprechend erhöht werden muß. Dies bewirkt jedoch auch eine zunehmende Erwärmung der Kappe bzw. des distalen Endes des Lichtleiters. Außerdem ist es mit den bekannten Einrichtungen nicht möglich innerhalb eines Hohlorganes mit deutlich größerem Durchmesser als der Kappe bzw. der Lichtleitfaser die Laserstrahlung gerichtet auf kleine Bereiche der Innenwand zu bringen.

Es ist daher Aufgabe der Erfindung eine Einrichtung zur zirkumferenziellen Bestrahlung von Objekten zu schaffen, mit welcher insbesondere Laserlicht gerichtet und mit weiter vergrößerter radialer Komponente als bisher abgestrahlt werden kann. Diese Aufgabe wird durch eine nach den kennzeichnenden Merkmalen des Patentanspruchs 1 ausgebildete Einrichtung gelöst.

Aus "Handbook of Optics", Walter G. Driscoll, 1978, Seite 13-7 bis 13-8 ist die Eigenschaft von Lichtleitfasern, daß ein nicht axial eingekoppeltes Strahlenbündel aus der Lichtleitfaser in Form eines Kegelmantels austritt, bekannt. Die am distalen Ende der Lichtleitfaser austretende Strahlung hat also eine deutliche radiale Komponente und bleibt dennoch gerichtet. Erfindungsgemäß wird diese radiale Komponente nun dadurch vergrößert, daß um das distale Ende der Lichtleitfaser herum eine Grenzfläche zweier transparenter Medien mit unterschiedlichem Brechungsindex geschaffen wird, auf die die Strahlung schräg auftrifft. Dies läßt sich in einfacher Weise dadurch realisieren, daß um das distale Ende eine Kappe ähnlich derjenigen aus "Laser in Surgery and Medicine" geschoben wird, die jedoch keine Maßnahmen zur diffusen Streuung des Lichtes aufweist, sondern möglichst klar und transparent ist. Je nach Anwendungsfall ist dann dafür zu sorgen, daß die an die Innenwand und an die Außenwand der Kappe angrenzenden Medien unterschiedliche Brechungsindizes haben, was beispielsweise dadurch bewirkt wird, daß der Hohlraum innerhalb der Kappe mit Luft und die Außenwand der Kappe entweder mit dem zu bestrahlenden Gewebe, der Gewebeflüssigkeit oder mit einer Spülflüssigkeit in Berührung steht.

Dadurch, daß die optische Strahlung aus der Lichtleitfaser trotz radialer Komponente gerichtet austritt, kann die erfindungsgemäße Einrichtung mit besonderem Vorteil im zentralen Lumen eines distal geschlossenen Ballonkatheters angeordnet sein, der zumindest im Bereich des Ballons aus einem lichtdurchlässigen Material hergestellt ist, wobei das Lumen des Katheters und der Ballon mit den optisch unterschiedlich dichten transparenten Medien füllbar ist. Hierdurch wird eine gleichzeitige Ballondilatation und Laserbehandlung beispielsweise einer Gefäßwand möglich. Die Lichtleitfaser ist im Kathetersystem beweglich und erlaubt damit eine Laserkoagulation der Gefäßwand im Bereich des Ballons bzw. auf der gesamten Länge des Katheters. Die distale Spitze des Ballonkatheter-Lichtleitersystems kann konisch und länger gestaltet werden, um damit eine Modifizierung des Brechungswinkels durch Beugung an der Grenzfläche von Luft zu Ballonfüllungsmedium zu erreichen. Die Lichtleitfaser kann am distalen Ende der Ummantelung eine röntgendichte Markierung und in einem bestimmten Abstand davon weiter proximal eine Markierung haben, wobei dieser Abstand dem Abstand zwischen der distalen Markierung und den für den jeweiligen Ballondurchmesser im gefüllten Zustand bestimmbaren Auftreffort des Strahlungskegels auf der Gefäßwand entspricht. Dadurch kann der behandelnde Arzt ein gutes Gefühl dafür entwickeln, in welchem Abstand vom distalen Ende der Lichtleitfaser der Kegelmantel der optischen Strahlung auf die zu behandelnde Wandung auftrifft.

Indem die lichtdurchlässige Kappe auf Teilen ihres Umfanges verspiegelt ist, wird es möglich, das zu behandelnde Hohlorgan oder dergleichen nicht auf den gesamten Umfang mit der optischen Strahlung zu behandeln. Dadurch kann also eine umfangsmäßige Selektion des zu behandelnden Bereiches erfolgen.

Wenn die Kappe mit einer röntgendichten Drahtspitze am distalen Ende verlängert ist, gelingt es, die Lichtleitfaser mit der Drahtspitze als Führungsdraht für ein herkömmliches Ballonkathetersystem zu verwenden, wie dies bisher nicht möglich war. Nach bisheriger Technik pflegte man zunächst einen Führungsdraht in das zu behandelnde Blutgefäß einzulegen, um dann ohne weitere Einfädelprobleme Ballonkatheter und dergleichen an diesem Führungsdraht entlang einzuführen bzw. wieder herauszunehmen. Durch die Hinzufügung einer Drahtspitze am distalen Ende der mit einer erfindungsgemäßen Kappe versehenen Lichtleitfaser gelingt es nunmehr, solche Lichtleitfasern selbst als Führungsdraht für derartige Applikationen einzusetzen. Da diese Drahtspitze zudem röntgendicht ist, ergibt sich der große Vorteil, daß man auf dem Röntgenschirm genau beobachten kann, wo sich das distale Ende der Lichtleitfaser befindet.

Es ist weiterhin möglich, die Lichtleitfaser auf deren Ummantelung oder auch auf der Kappe, die gegebenenfalls schlauchartig bis zum proximalen Ende verlängert ist, mit einer oder mehreren röntgendichten Markierungen zu versehen, so daß die Lichtleitfaser ohne Schwierigkeiten auf dem Röntgenschirm beobachtet werden kann. Diese Markierungen können entweder einzelne Marken sein oder ein sich in Längsrichtung der Lichtleitfaser erstreckender durchgehender Streifen oder dergleichen sein.

Insbesondere zur Behandlung arrhythmogener Strukturen im Herzmuskel (Myokard) ist es vom großen Vorteil, wenn die Lichtleitfaser am distalen Ende bzw. auf der Oberfläche der Kappe zumindest eine Elektrode aufweist, mit welcher die Auswirkungen der koagulierenden Strahlung auf das Myokard unmittelbar gemessen werden können.

Die Erfindung soll im folgenden anhand der in den Figuren teilweise schematisch dargestellten Ausführungsbeispielen näher beschrieben werden. Es zeigen:
- Fig. 1: den Strahlenverlauf innerhalb einer Lichtleitfaser bei schräger Einkopplung,
- Fig. 2: das distale Ende einer Lichtleitfaser mit Maßnahmen zur Erweiterung des Strahlenkegels,
- Fig. 3: eine Lichtleitfaser mit Kappe zur Bildung einer optischen Grenzfläche,
- Fig. 4: eine Lichtleitfaser mit tropfenförmiger Glaskappe,
- Fig. 5: eine Lichtleitfaser mit Kappe und Führungsdraht und
- Fig. 6: eine Lichtleitfaser, die in einem Ballonkatheter eingesetzt ist, wobei die optische Grenzfläche von der inneren Wandung des Ballonkatheters ausgebildet wird.

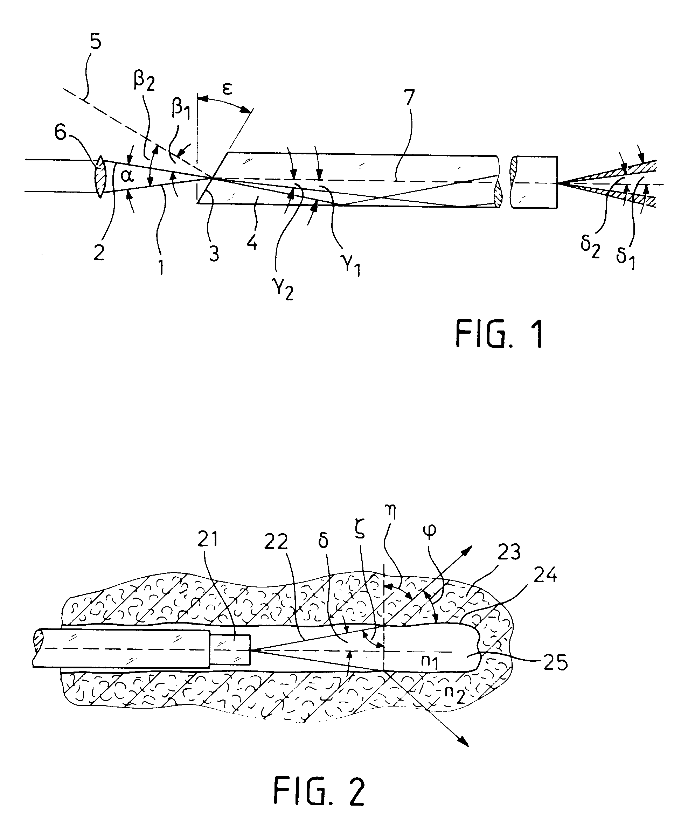

Zur Erklärung der Funktionsweise der in Fig. 1 dargestellten Einrichtung werden folgende Symbole verwendet:
- ε: Winkel unter dem das proximale Ende der Lichtleitfaser angeschliffen ist,
- α: Konvergenzwinkel mit der eine einzukoppelnde Laserstrahlung auf das proximale Ende der Lichtleitfaser fokussiert wird,
- β₁: Winkel des Randstrahls 1 am proximalen Ende der Lichtleitfaser bezogen auf die Flächennormale der Stirnfläche der Lichtleitfaser,
- β₂: Winkel des Randstrahls 2 (analog zu β₁),
- γ₁: Winkel des Randstrahls 1 innerhalb der Lichtleitfaser bezogen auf die Faserlängsachse,
- γ₂: Winkel des Randstrahls 2 (analog zu γ₁),
- γₘₐₓ: maximal zulässiger Strahlungswinkel innerhalb der Lichtleitfaser bezogen auf die Faserlängsachse (Bedingung für Totalreflexion),
- δ₁: Austrittswinkel des Randstrahls 1 am distalen Ende der Lichtleitfaser bezogen auf die Faserlängsachse,
- δ₂: Austrittswinkel des Randstrahls 2 (analog zu δ₁).
- NA: Numerische Apertur der Lichtleitfaser und
- n_{L}: Brechungsindex des Kerns der Lichtleitfaser.

Unter Verwendung der o.g. Symbole gelten für die Anordnung gemäß Fig. 1 folgende Gleichungen:
- γₘₐₓ: = arc sin NA/n_{L} (1)
- γ₁: = ε - arc sin (1/n_{L} x sinβ₁) (2)
- γ₂: = ε - arc sin (1/n_{L} x sinβ₂) (3)
- δ₁: = arc sin (n_{L} x sinγ₁) (4)
- δ₂: = arc sin (n_{L} x sinγ₂) (5)

Mit diesen Gleichungen lassen sich die Abstrahlcharakteristik der Lichtleitfaser und vor allem der Öffnungswinkel des Kegelmantels der austretenden Strahlung berechnen. Bei kleinem Konvergenzwinkel α der einzukoppelnden Laserstrahlung kann dabei die Divergenz der austretenden Strahlung innerhalb des Kegelmantels, also die Unterschiede zwischen δ₁ und δ₂ vernachlässigt werden.

Die Einkopplung der von einem nicht dargestellten Laser ausgehenden Strahlung erfolgt über eine Linse 6, welche die Strahlung auf die Stirnfläche 3 der Lichtleitfaser 4 fokusiert. Die Stirnfläche 3 ist dabei unter einem bestimmten Winkel ε angeschliffen, so daß die optische Achse von Laser und Linse 6 in der Faserlängsachse 7 liegen kann und dennoch eine "schräge" Einkopplung erreicht wird; dies erleichtert den mechanischen Aufbau und die Justierung erheblich gegenüber solchen Anordnungen aus "Handbook of Optics". Der Winkel ε sollte so groß wie möglich gewählt werden, darf jedoch die für die numerische Apertur der Lichtleitfaser geltende Bedingung gemäß Gleichung (1) nicht überschreiten. Die Grenze für den Winkel ε ist gemäß Gleichung (2) bzw. (3) vom Konvergenzwinkel α und dem Brechungsindex des Kerns der Lichtleitfaser 4 abhängig.

Eine Vergrößerung des Öffnungswinkels δ des Kegelmantels der austretenden Strahlung läßt sich gemäß Fig. 2 dadurch erzielen, daß die aus der Lichtleitfaser 21 austretende Strahlung 22 auf eine zylinderförmige Grenzfläche 24 trifft, wobei die Medien 23 und 25 außerhalb bzw. innerhalb der zylindrischen Grenzfläche 24 transparent sind, jedoch mit deutlich unterschiedlichen Brechungsindizes; das Medium 23 sollte einen möglichst hohen Brechungsindex n₂ und das Medium 25 einen möglichst niedrigen Brechungsindex n₁ aufweisen. Für die medizinische Anwendung bietet sich an, daß das Medium 25 Luft oder ein Inertgas ist und das Medium 23 Wasser bzw. eine Kochsalzlösung darin ist. Die aus der Lichtleitfaser 21 kegelmantelförmig austretende Strahlung wird dann an dieser zylindrischen Grenzfläche 24 derart gebrochen, daß eine Vergrößerung des Öffnungswinkels δ des Kegelmantels bewirkt wird. Hierbei gelten folgende Bedingungen:
- δ+ζ: = 90 Grad (6)
- η+ψ: = 90 Grad (7)
- sinζ/sinη: = n₂/n₁ (8)
- V: = ψ/δ (Vergrößerung des Öffnungswinkels) (9)

Für einen typischen, in der Medizintechnik in Verbindung mit Neodym-YAG-Lasern verwendeten Lichtleiter mit einer numerischen Apertur von NA = 0,3 und einem Brechungsindex des Quarzglaskerns von n = 1,45 läßt sich bei einem Anschliff der Stirnfläche 3 um einen Winkel ε = 24° ein mittlerer Öffnungswinkel δ = 22° des austretenden Strahlungskegelmantels erzielen. Bei Anbringung einer koaxialen, zylindrischen Grenzfläche 24 gemäß Fig. 2 mit Wasser für das Medium 23 und Luft für das Medium 25 läßt sich etwa eine Vervierfachung des Öffnungswinkels erzielen, so daß die Abstrahlung etwa unter einem Winkel von 43° zur Faserachse erfolgt.

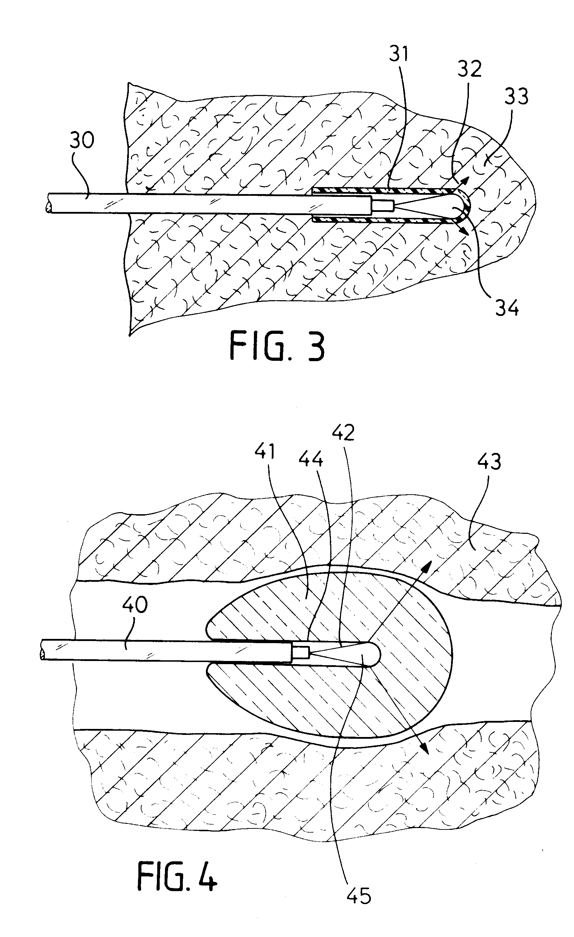

Bei dem in Fig. 3 dargestellten Ausführungsbeispiel ist das distale Ende der Lichtleitfaser 30 mit einer transparenten Teflonkappe 31 umgeben, die mit dem Faserende einen luftgefüllten Hohlraum 34 einschließt. Das distale Ende der Lichtleitfaser 30 mit der Kappe 31 steckt tief in einem zu behandelnden Gewebe 33. Die kegelmantelförmig aus dem Lichtleiter 30 austretende Laserstrahlung 32 wird hier an der Grenzfläche Luft-Gewebe bzw. Gewebeflüssigkeit entsprechend den optischen Eigenschaften gebrochen; die optischen Eigenschaften der Teflonkappe 31 können unberücksichtigt bleiben. Mit einer solchen Anordnung lassen sich z.B. tief liegende arrhythmogene Strukturen im Myokard koagulieren.

Bei dem in Fig. 4 dargestellten Ausführungsbeispiel ist am distalen Ende einer Lichtleitfaser 40 eine tropfen- bzw. olivenförmige Kappe 41 aus Glas angeordnet, die eine Eintrittsbohrung 44 für die Lichtleitfaser 40 aufweist. Das distale Ende der Lichtleitfaser 40 schließt mit der Bohrung 44 wiederum einen luftgefüllten Hohlraum 45 ein, so daß die aus der Lichtleitfaser 40 austretende Laserstrahlung 42 an der Wandung der Bohrung 44 erneut gebrochen und somit der Strahlungskegel noch weiter aufgeweitet wird. Mit einer derartigen Einrichtung lassen sich z.B. Tumore 43 im Ösophagus und in den Bronchien koagulieren.

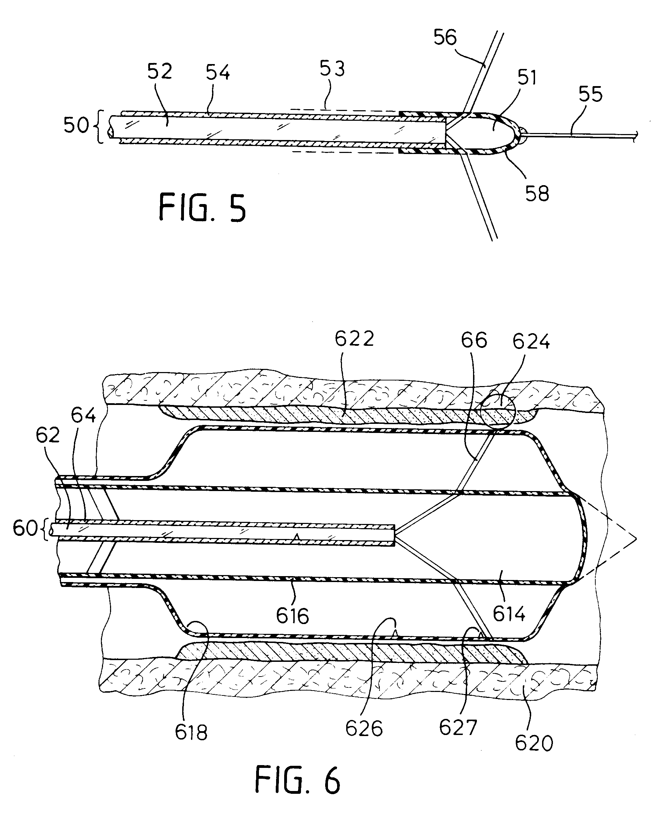

Fig. 5 zeigt den herkömmlichen Faserkern 52 einer üblichen Lichtleitfaser 50 mit einem Mantel 54, wobei das Laserlicht derart in den Faserkern eingekoppelt werden kann, daß es am distalen Ende kegelmantelförmig austritt. Indem die Lichtleitfaser 50 mit einer Kappe 58 aus lichtdurchlässigem Material versehen wird, die einen Hohlraum 51 definiert, der mit einem geeigneten Gas gefüllt sein kann, bildet die Kappe 58 eine optische Grenzfläche zwischen dem optisch dünneren Medium in dem Hohlraum 51 und dem optisch dichteren Medium außen um die Lichtleitfaser herum, welches beispielsweise in das zu behandelnde Hohlorgan eingefüllt worden ist. Bei einem solchen optisch dichteren Medium kann es sich ohne weiteres um eine röntgendichte, lichtdurchlässige Kontrastflüssigkeit handeln. Durch eine derartige Ausgestaltung der Lichtleitfaser mit der eine optische Grenzfläche definierenden Kappe 58 wird der Kegelmantel wie in Fig. 3 relativ aufgeweitet mit der Folge, daß das Laserlicht 56 möglichst weitgehend radial auf die zu behandelnde Wandung oder dergleichen auftrifft.

Die Kappe 58 kann bis zum proximalen Ende der Lichtleitfaser 50 schlauchartig verlängert sein (53) und z.B. innen mit Druckgas gespült bzw. gekühlt werden.

In besonders vorteilhafter Weise ist die Kappe 58 an ihrem vorderen Ende mit einer Drahtspitze 55 versehen, die ausreichend lang ausgestaltet wird, um die Lichtleitfaser selbst als Führungsdraht, beispielsweise für ein herkömmliches Ballonkathetersystem, einsetzen zu können, bei dem der Ballonkatheter über einen Führungsdraht geschoben wird, nachdem letzterer in dem zu untersuchenden Gefäß oder Organ plaziert worden ist.

In Fig. 6 ist die Lichtleitfaser im Zusammenhang mit einem Ballonkatheter beim Einsatz in einer Gefäßwandung gezeigt, bei der Ablagerungen 622 auf der Innnenseite der Gefäßwandung 620 durch den Ballon 618 des Ballonkatheters 616 dilatiert werden. Im Bereich 624 findet eine Laserkoagulation statt, und zwar durch den in der erfindungsgemäßen Weise aufgeweiteten Laserlicht-Kegelmantel 66, der aus dem Faserkern 62 der Lichtleitfaser 60 austritt. Dieser ist im zentralen Lumen 614 des Ballonkatheters 616 angeordnet, der am distalen Ende geschlossen ist, und zwar entweder wie dargestellt mit einer mehr oder weniger glatten Wandung, oder mittels einer beispielsweise spitz zulaufenden Wandung, wodurch eine optische Grenzfläche geschaffen wird, die nicht mehr koaxial zur Längsachse des Katheters verläuft. Das zentrale Lumen 614 des Ballonkatheters muß mit einem optisch dünneren Medium gefüllt werden als der Ballon 618 des Ballonkatheters, um die gewünschte Aufspreizung des Laserlicht-Kegelmantels zu erreichen. Der Ballon 618 weist zwei röntgendichte Markierungen 626 und 627 auf, die zum einen in Höhe des distalen Endes der Lichtleitfaser und zum anderen am ringförmigen Auftreffbereich des Strahlungskegels 66 auf die Ballonwand angebracht sind. Durch die in Fig. 6 dargestellte Kombination einer seitlich abstrahlenden Lichtleitfaser mit einem Ballonkatheter wird erreicht, daß gleichzeitig dilatiert und koaguliert werden kann. Dies war bisher nicht möglich.

## Patentansprüche

1. Einrichtung zur zirkumferenziellen medizinischen Bestrahlung von Objekten, insbesondere von Gefäßen, Hohlorganen und soliden Geweben mittels optischer Strahlung hoher Intensität, welche über eine flexible Lichtleitfaser (4) geführt wird, in die die optische Strahlung unter einem vorgegebenen Winkel (β₁, β₂) zur Flächennormalen (5) der Stirnfläche (3) der Lichtleitfaser (4) eingekoppelt wird derart, daß sie am distalen Ende der Lichtleitfaser (4) in Form eines Kegelmantels (22) gerichtet austritt, **dadurch gekennzeichnet,** daß zur Vergrößerung des Öffnungswinkels (δ) des austretenden Strahlungskegelmantels (22) Mittel (31, 41, 58) vorgesehen sind, die um das distale Ende der Lichtleitfaser (4) herum eine Grenzfläche (24) zweier transparenter Medien (23, 25) mit unterschiedlichem Brechungsindex (n₂, n₁) schaffen, auf die der Strahlungskegelmantel (22) schräg auftrifft.

2. Einrichtung nach Anspruch 1 **dadurch gekennzeichnet,** daß die Stirnfläche (3) am proximalen Ende der Lichtleitfaser (4) unter einem von 90° zur Faserachse (7) abweichenden Winkel (ε) angeschliffen ist und die Einkopplung der optischen Strahlung in Richtung der Faserachse (7) erfolgt.

3. Einrichtung nach Anspruch 1 oder 2 **dadurch gekennzeichnet,** daß das distale Ende der Lichtleitfaser (30, 50) eine im wesentlichen zylindrische Kappe (31, 58) mit transparenter Zylinderwand aufweist, wobei zwischen der Kappe (31, 58) und der Stirnfläche der Lichtleitfaser ein Hohlraum (34, 51) vorgesehen ist.

4. Einrichtung nach Anspruch 3 **dadurch gekennzeichnet,** daß der Hohlraum (34) mit einem gasförmigen Medium gefüllt ist.

5. Einrichtung nach Anspruch 3 oder 4 **dadurch gekennzeichnet,** daß die Kappe (58) bis zum proximalen Ende der Lichtleitfaser (50) schlauchartig (53) verlängert ist.

6. Einrichtung nach einem der Ansprüche 3 bis 5 **dadurch gekennzeichnet,** daß die Kappe auf Teilen ihres Umfanges verspiegelt ist.

7. Einrichtung nach einem der Ansprüche 3 bis 5 **dadurch gekennzeichnet,** daß die Kappe (58) mit einer röntgendichten Drahtspitze (55) am distalen Ende verlängert ist.

8. Einrichtung nach einem der Ansprüche 1 oder 2 **dadurch gekennzeichnet,** daß das distale Ende der Lichtleitfaser (60) in einem zentralen Lumen (614) eines distal geschlossenen Ballonkatheters (616) angeordnet ist, der zumindest im Bereich des Ballons (618) aus einem transparenten Material hergestellt ist, wobei das Lumen (614) des Katheters und der Ballon (618) mit den optisch unterschiedlich dichten, transparenten Medien füllbar ist.

9. Einrichtung nach einem der Ansprüche 3 bis 8 **dadurch gekennzeichnet,** daß eine oder mehrere röntgendichte Markierungen (626, 627) auf dem Ballon oder der Kappe (58, 618) vorgesehen sind.

## Claims

1. Device for circumferential medical radiation of objects, in particular of vessels, hollow elements amd solid fabrics, by means of high intensity optical radiation which is directed via a flexible fibre optic (4) into which the optical radiation is coupled at a specified angle (β₁, β₂) to a surface normal (5) of an end surface (3) of a fibre optic (4), so that it exits at the distal end of the fibre optic (4) oriented in the form of a conical lobe (22), **characterised in that** means (31, 41, 58) are provided for enlarging the aperture angle (δ) of the exiting concal radiation lobe (22), which establish around the distal end of the fibre optic (4) a defining surface (24) of two transparent media (23, 25) of different refraction index (n₂, n₁) which are hit transversely by the conical radiation lobe (22).

2. Device according to claim 1, **characterised in that** the end surface (3) at the proximal end of the fibre optic (4) is ground at an angle (ε) which deviates by 90° from the fibre axis (7), and that the optical radiation is coupled in the direction of the fibre axis (7).

3. Device according to claim 1 or 2, **characterised in that** the distal end of the fibre optic (30, 50) comprises a substantially cylindrical cap (31, 58) having a transparent cylinder wall, thus providing a hollow space (34, 51) between the cap (31, 58) and the end surface of the fibre optic.

4. Device according to claim 3, **characterised in that** the hollow space (34) is filled with a gaseous medium.

5. Device according to claim 3 or 4, **characterised in that** the cap (58) is hose-like (53) extended to the proximal end of the fibre optic (50).

6. Device according to one of claims 3 to 5, **characterised in that** the cap is translucent in parts of its circumference.

7. Device according to one of claims 3 to 5, **characterised in that** the cap (58) is extended by means of an x-ray proof wire tip (55) at the distal end.

8. Device according to one of claims 1 or 2, **characterised in that** the distal end of the optical fibre (60) is arranged in a central lumen (614) of a distally sealed balloon catheter (616) which is made, at least in the area of the balloon (618), of a transparent material, in which respect the lumen (614) of the catheter and the balloon (618) can be filled with transparent media of different optical density.

9. Device according to one of claims 3 to 8, **characterised in that** one or several x-ray proof markings (626, 627) are provided on the balloon or on the cap (58, 618).

## Revendications

1. Dispositif pour l'irradiation médicale circonférentielle d'objets, en particulier de vaisseaux, d'organes creux et de tissus solides au moyen d'un rayonnement optique de forte intensité qui est amené par l'intermédiaire d'une fibre optique (4) dans laquelle le rayonnement optique est introduit sous un angle (β₁, β₂) prédéterminé par rapport à la normale (5) à la surface frontale (3) de la fibre optique (4) de manière telle que ledit rayonnement optique sorte à l'extrémité distale de la fibre optique (4) sous la forme d'une enveloppe conique (22), caractérisé par le fait que des moyens (31, 41, 58) sont prévus pour augmenter l'angle d'ouverture (δ) de l'enveloppe conique (22) du rayonnement sortant, lesquels moyens forment tout autour de l'extrémité distale de la fibre optique (4) une interface (24) de deux milieux transparents (23, 25) à indice de réfraction (n₂, n₁) différent sur laquelle l'enveloppe conique (22) du rayonnement arrive sous un angle d'inclinaison.

2. Dispositif selon la revendication 1, caractérisé par le fait que la surface frontale (3) de l'extrémité proximale de la fibre optique (4) est polie suivant un angle (ε) qui s'écarte de 90° par rapport à l'axe (7) de la fibre et que le rayonnement optique est introduit suivant la direction de l'axe (7) de la fibre.

3. Dispositif selon la revendication 1 ou 2, caractérisé par le fait que l'extrémité distale de la fibre optique (30, 50) comporte une coiffe (31, 58) sensiblement cylindrique dont la paroi cylindrique est transparente, une cavité (34, 51) étant prévue entre la coiffe (31, 58) et la surface frontale de la fibre optique.

4. Dispositif selon la revendication 3, caractérisé par le fait que la cavité (34) est remplie d'un milieu gazeux.

5. Dispositif selon la revendication 3 ou 4, caractérisé par le fait que la coiffe (58) se prolonge sous forme de tube souple (53) jusqu'à l'extrémité proximale de la fibre optique (50).

6. Dispositif selon l'une des revendications 3 à 5, caractérisé par le fait que la coiffe est réfléchissante sur des parties de sa circonférence.

7. Dispositif selon l'une des revendications 3 à 5, caractérisé par le fait que la coiffe (58) se prolonge à son extrémité distale par une pointe en fil (55) imperméable aux rayons X.

8. Dispositif selon l'une des revendications 1 ou 2, caractérisé par le fait que l'extrémité distale de la fibre optique (60) est insérée dans une lumière (614) d'un catheter (616) à ballon fermé côté distal qui, dans la région du ballon (618) au moins, est réalisé en un matériau transparent, la lumière (614) du catheter et le ballon (618) pouvant être remplis avec les milieux transparents de densité optique différente.

9. Dispositif selon l'une des revendications 3 à 8, caractérisé par le fait qu'il est prévu sur le ballon ou sur la coiffe (58, 618) un ou plusieurs repères (626, 627) imperméables aux rayons X.
